(19) **Europäisches Patentamt European Patent Office Office européen des brevets**

(11) **EP 2 181 324 B1**

(12) **FASCICULE DE BREVET EUROPEEN**

(45) Date de publication et mention
de la délivrance du brevet:
**10.08.2011 Bulletin 2011/32**

(21) Numéro de dépôt: **07788896.4**

(22) Date de dépôt: **15.06.2007**

(51) Int Cl.:
***G01N 29/06*** *(2006.01)*

(86) Numéro de dépôt international:
**PCT/FR2007/001002**

(87) Numéro de publication internationale:
**WO 2007/144520 (21.12.2007 Gazette 2007/51)**

(54) **PROCEDE DE MESURE DE PROPRIETES VISCOELASTIQUES DE TISSUS BIOLOGIQUES METTANT EN OEUVRE UN TRANSDUCTEUR ULTRASONORE**

VERFAHREN ZUR MESSUNG VISKOELASTISCHER EIGENSCHAFTEN VON BIOLOGISCHEM GEWEBE UNTER EINSATZ EINES ULTRASCHALLWANDLERS

METHOD OF MEASURING VISCOELASTIC PROPERTIES OF BIOLOGICAL TISSUE EMPLOYING AN ULTRASONIC TRANSDUCER

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **15.06.2006 FR 0605342**

(43) Date de publication de la demande:
**05.05.2010 Bulletin 2010/18**

(73) Titulaire: **EchoSens S.A.**
**75013 Paris (FR)**

(72) Inventeurs:
• **SANDRIN, Laurent**
  **92240 l'Hay les Roses (FR)**
• **MIETTE, Véronique**
  **F-94800 Villejuif (FR)**

(74) Mandataire: **Camus, Olivier Jean-Claude**
  **Cabinet Camus Lebkiri**
  **10 rue de la Pépinière**
  **75008 Paris (FR)**

(56) Documents cités:
**WO-A2-2004/021888     FR-A- 2 869 521**
**FR-A1- 2 843 290**

**Description**

La mesure de propriétés viscoélastiques, dénommées PV par la suite, de tissus biologiques peut permettre le diagnostic, le dépistage ou le suivi de traitements relatifs à, par exemple, des organes tels que le foie, la peau ou des vaisseaux sanguins.

Afin d'effectuer une mesure non invasive respectant l'intégrité d'un organe considéré, il est connu d'utiliser un dispositif émettant une onde de cisaillement à basse fréquence dans les tissus biologiques de cet organe puis de mesurer, au moyen d'acquisitions ultrasonores, la réponse du tissu biologique à cette onde de cisaillement. Un tel procédé est décrit, par exemple, dans la demande de brevet FR 2869521 déposée le 3 mai 2004 au nom de la société Echosens Société Anonyme.

On connaît un procédé de mesure des PV de tissus biologiques utilisant un dispositif compris dans une des trois catégories décrites ci-dessous :

- Une première catégorie de dispositif présente un transducteur composé d'un seul élément transformant des ondes ultrasonores réfléchies par les tissus considérés en signaux électriques. Dans ce cas, un tel transducteur met en oeuvre un unique faisceau ultrasonore, ce qui ne permet pas de mesurer les PV d'organes hétérogènes.

L'utilisation d'un tel dispositif est ainsi limitée à une mesure moyenne des PV pour l'ensemble d'un organe, une telle mesure moyenne ne permettant pas d'effectuer des mesures locales de PV en vue de détecter, par exemple, une pathologie localisée dans cet organe.

- Une seconde catégorie de dispositif présente un transducteur comprenant un alignement d'éléments transformant des ondes ultrasonores réfléchies par les tissus considérés en signaux électriques.

Dans ce cas, un tel transducteur ne reçoit que des ondes ultrasonores réfléchies relatives à un plan à deux dimensions. Or l'obtention des PV d'un plan de tissu biologique nécessite des informations volumiques à trois dimensions, notamment en élévation, c'est-à-dire selon une direction orthogonale au plan considéré.

De ce fait, l'utilisation d'un tel dispositif ne permet pas de mesurer quantitativement les PV du tissu considéré. En d'autres termes, un tel dispositif ne fournit uniquement que des informations qualitatives locales qui sont soumises à des artéfacts dus à l'approximation effectuée en négligeant les variations des déformations des tissus engendrées en élévation.

- Finalement, une troisième catégorie de dispositif, décrite dans la demande de brevet FR 2869521 précédemment citée, utilise un transducteur comprenant quatre éléments circulaires non alignés transformant des ondes ultrasonores réfléchies par les tissus considérés en signaux électriques..

Ce type de dispositif vise notamment à fournir une mesure quantitative des PV de tissus biologiques. Mais il présente l'inconvénient d'utiliser des éléments dont les dimensions importantes sont strictement imposées par les caractéristiques du faisceau ultrasonore souhaité. A titre d'exemple, dans le foie, avec un transducteur ultrasonore de fréquence centrale 3.5 Mhz, cela nécessite des éléments de diamètre 7 mm pour pouvoir effectuer une mesure à des profondeurs comprises entre 20 et 80 mm.

L'invention résout au moins un des problèmes précédemment indiqué en fournissant un procédé de mesures des PV d'un tissu biologique qui permet de faire des mesures quantitatives de PV locales tout en ayant une résolution satisfaisante.

De fait, la présente invention concerne un procédé de mesure de PV de tissus biologiques mettant en oeuvre un transducteur ultrasonore muni d'éléments transformant des ondes ultrasonores réfléchies par ces tissus biologiques en signaux électriques, différents éléments étant groupés pour former des sous-ouvertures telles que l'acquisition des signaux électriques issus des éléments d'une même sous-ouverture est effectuée simultanément, chacune de ces sous-ouvertures étant interceptée par un axe de propagation d'ondes ultrasonores en un centre acoustique, caractérisé en ce qu'au moins un même élément appartient à au moins deux sous-ouvertures différentes et en ce qu'un centre acoustique est entouré par au moins trois autres centres acoustiques non alignés.

Il convient de noter qu'un axe de propagation d'ondes ultrasonores correspond à l'axe sur lequel la distribution d'énergie est maximale.

Un tel procédé présente de nombreux avantages. De fait, l'utilisation d'au moins un élément commun à différentes sous-ouvertures permet de réduire la distance entre les centres acoustiques des différentes sous-ouvertures sans réduire la surface d'émission et de réception des ondes ultrasonores, ce qui permet d'effectuer des mesures de PV avec une résolution accrue.

De plus, le fait que le centre acoustique d'une sous-ouverture soit entouré par au moins trois centres acoustiques non alignés permet d'obtenir des données volumiques nécessaires pour être en mesure de calculer des PV locaux, comme détaillé ultérieurement.

Finalement, la mise en oeuvre conjointe des deux dispositions indiquées ci-dessus permet d'utiliser un nombre réduit d'éléments transformateurs d'ondes ultrasonores en signaux électriques tout en ayant une résolution améliorée par rapport à l'art antérieur pour effectuer des mesures locales de PV.

Dès lors, le coût et la complexité d'un procédé de mesure de PV conforme à l'invention sont réduits alors même que ce procédé peut effectuer une mesure locale, voire quantitative, des PV des tissus d'un organe avec une résolution suffisante pour mesurer des PV localisées aptes à identifier, par exemple, une tumeur dans un organe.

Dans une réalisation, le procédé comprend l'étape d'utiliser différentes sous-ouvertures simultanément, par exemple en utilisant un même élément dans des sous-ouvertures utilisées simultanément.

L'utilisation simultanée de sous-ouvertures permet d'obtenir une cadence d'acquisition des données ultrasonores plus rapide. En effet l'utilisation d'au moins un élément commun à différentes sous-ouvertures est comparable à deux tirs simultanés pour ce même élément ce qui permet d'obtenir une cadence supérieure à ce qui serait possible si les signaux provenant de chaque sous-ouverture était formés séquentiellement.

La formation de voies pour une sous-ouverture donnée correspond à la sommation - avec ou sans décalages temporels (loi de retard) - des signaux issus des différents éléments constituant cette sous-ouverture.

Cette sommation peut être réalisée selon plusieurs méthodes ; à titre d'exemple on peut citer la sommation des signaux analogiques électriques issus des différents éléments, la sommation dans un composant électronique après numérisation, la sommation logicielle dans un programme informatique.

De fait, un balayage électronique séquentiel de sous-ouvertures est remplacé par au moins une acquisition parallèle, c'est-à-dire simultanée, pour ces sous-ouvertures.

Dans une réalisation, le procédé comprend l'étape d'entraîner les tissus en mouvement, cette mise en mouvement pouvant s'effectuer de façon manuelle ou automatique.

Dans une réalisation, le procédé comprend l'étape de former des sous-ouvertures de telle sorte que les centres acoustiques de ces sous-ouvertures forment une grille présentant un maillage triangulaire, par exemple équilatéral.

Selon une réalisation, le procédé comprend l'étape de former des sous-ouvertures de telle sorte qu'une sous-ouverture soit entièrement délimitée par la surface d'autres sous-ouvertures.

Dans une réalisation, le procédé comprend l'étape supplémentaire de former les sous-ouvertures de telle sorte qu'un centre acoustique soit entouré par six centres acoustiques équidistants.

L'invention concerne également un dispositif de mesure des PV de tissus biologiques muni d'un transducteur ultrasonore comprenant des éléments qui transforment des ondes ultrasonores réfléchies par ces tissus biologiques en des signaux électriques, caractérisé en ce que des éléments sont situés à une distance, mesurée entre leurs centres, comprise entre 0,5 et 5 mm, préférentiellement entre 2 et 5 mm.

Une telle distance entre les éléments du transducteur est obtenue grâce à la mise en oeuvre d'un procédé conforme à l'une des réalisations précédemment décrites. Aussi, un tel dispositif présente l'avantage de permettre de mesurer des PV avec une résolution satisfaisante pour un faible coût compte tenu du nombre réduit d'éléments requis pour la mise en oeuvre de l'invention.

Selon une réalisation, le dispositif comprend des moyens pour acquérir simultanément des signaux électriques reçus par une pluralité d'éléments regroupés en une sous-ouverture et des moyens pour effectuer la formation de voies de transmission de signaux électriques correspondant à plusieurs sous-ouvertures simultanées présentant au moins un élément commun.

Dans une réalisation, le dispositif comprend des moyens pour que le centre d'au moins une sous-ouverture soit entourée par au moins trois centres acoustiques non alignés entre eux.

Selon une réalisation, le dispositif comprend au moins 19 éléments hexagonaux ou au moins 24 éléments triangulaires équilatéraux.

Dans une réalisation, le dispositif comprend des éléments ayant la forme d'un polygone, par exemple un hexagone, un carré, un losange ou un triangle, ou d'un cercle.

L'invention concerne également une sonde munie d'un dispositif conforme à l'une des réalisations précédentes ainsi qu'un système muni d'un dispositif conforme à l'une des réalisations précédentes, ce système comprenant en outre des moyens pour effectuer un traitement par hyperthermie par ultrasons ou pour entraîner les tissus en mouvement.

Finalement, l'invention concerne des données issues d'un procédé, d'un dispositif, d'une sonde ou d'un système conforme à l'une des réalisations précédentes.

D'autres caractéristiques et avantages de l'invention apparaîtront à la lumière de la description d'une réalisation de l'invention effectuée ci-dessous, à titre illustratif et non limitatif, en faisant référence aux figures ci-jointes sur lesquelles :

- la figure 1 est un schéma d'un dispositif comprenant un transducteur conforme à l'invention,
- la figure 2 illustre des paramètres géométriques utilisées pour mesurer les PV d'un tissu biologique, et

- les figures 3, 4 et 5 illustrent différentes mises en oeuvre d'un procédé conforme à l'invention.

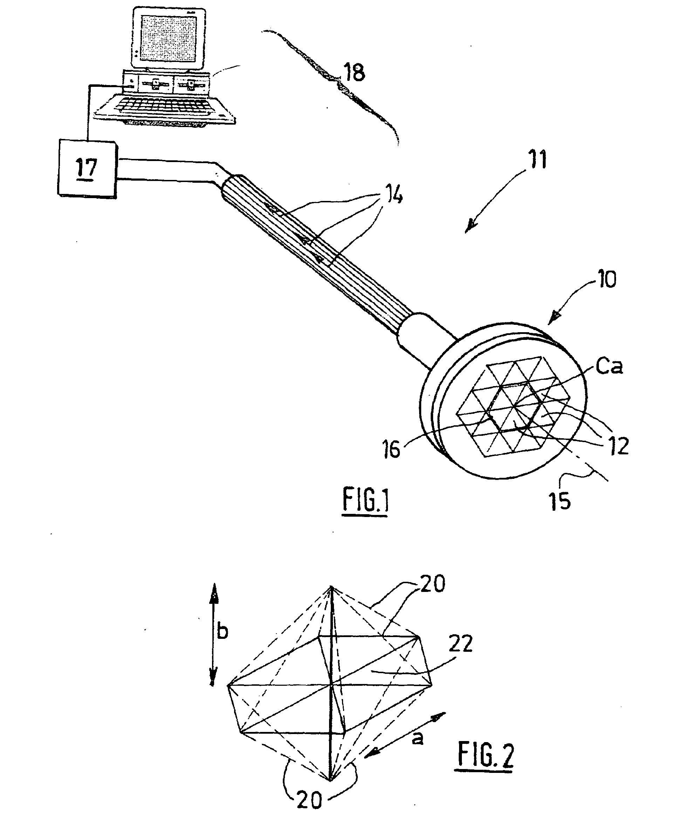

Un procédé de mesure des PV de tissus biologiques conforme à l'invention met en oeuvre une sonde 11 (figure 1) munie d'un transducteur ultrasonore 10 comprenant des éléments 12 qui transforment des ondes ultrasonores réfléchies par des tissus biologiques en des signaux électriques 14.

Ces signaux électriques 14 sont représentatifs de l'échogénéicité des tissus vis à vis des ondes ultrasonores, le terme échogénéicité dérivant du terme anglais « echogenicity ». Ainsi, un tissu est dit « hyperéchogène » lorsqu'il réfléchit fortement les ondes ultrasonores tandis qu'il est dit « hypoéchogène » lorsqu'il réfléchit faiblement les ondes ultrasonores.

Lors de la mise en oeuvre d'un procédé conforme à l'invention, différents éléments 12 forment des groupes dénommés sous-ouvertures tels que l'acquisition des signaux 14 issus des éléments 12 d'une même sous-ouverture 16 est effectuée simultanément.

Ainsi, lors de cette acquisition, la surface totale d'acquisition de la sous-ouverture 16 est la somme des surfaces de ses éléments 12.

A ce stade, il convient de noter que le transducteur 10 peut également être utilisé pour émettre des ondes ultrasonores destinées à être réfléchies par les tissus biologiques concernés. Dans ce cas, ces éléments 12 peuvent être groupés en sous-ouverture d'émission tandis que différentes sous-ouvertures 16 peuvent émettre simultanément.

Par ailleurs, une sous-ouverture 16 se caractérise par un axe 15 le long duquel se propage le faisceau d'ondes ultrasonores émises ou reçues par cette sous-ouverture 16, cet axe 15 interceptant la sous-ouverture en un point dénommé centre acoustique Ca. Pour des raisons de clarté, seul un axe 15, une sous-ouverture 16 et un centre Ca sont représentés sur la figure 1.

Conformément à l'invention, lors de la mesure des PV d'un tissu biologique, le procédé utilise différentes sous-ouvertures 16 de telle sorte qu'au moins un même élément 12 appartienne à au moins deux sous-ouvertures 16 différentes tandis que le centre acoustique Ca d'une sous-ouverture 16 est entouré par au moins trois autres centres acoustiques non alignés.

L'utilisation d'un élément 12 commun à différentes sous-ouvertures permet de réduire la distance entre les centres acoustiques des différentes sous-ouvertures et d'accroître la résolution, cette dernière pouvant être de l'ordre du millimètre, est obtenue alors même que le procédé utilise un nombre réduit et limité d'éléments, typiquement inférieur à trente.

Dans cet exemple compte tenu de l'utilisation d'acquisitions réalisées en parallèle sur plusieurs sous-ouvertures 16 simultanément, la cadence d'acquisition des données ultrasonores est très rapide en comparaison avec la cadence qui serait obtenue avec un balayage électronique séquentiel classique, c'est-à-dire utilisant des sous-ouvertures mises en oeuvre de manière successive.

La cadence ainsi obtenue est uniquement limitée par les temps de propagation des ondes ultrasonores et par la durée des échos de répétition. Cette cadence, typiquement de l'ordre de 4 KHz, et plus généralement comprise entre 100 Hz et 20 KHz, permet de mesurer les déformations volumiques engendrées dans les tissus par la propagation de l'onde de cisaillement à partir d'une seule excitation de cisaillement.

A ce stade, il convient de noter que cette excitation de cisaillement peut être effectuée à l'aide d'un vibreur externe à l'organe, d'une vibration organique générée par un organe du corps ou d'une vibration déclenchée à distance, par exemple en utilisant le principe de la pression de radiation.

Compte tenu de la très courte durée de propagation de l'onde de cisaillement, de l'ordre d'une centaine de millisecondes, l'acquisition de données volumiques et locales peut se faire sur des organes mobiles, par exemple le foie.

Un système 18 de mesure des PV comprenant la sonde 11 peut comprendre des moyens 17 pour acquérir simultanément des signaux reçus par une pluralité d'éléments regroupés en une pluralité de sous-ouvertures, lors de la phase de réception des ondes ultrasonores, et des moyens pour effectuer la formation de voies correspondant à plusieurs sous-ouvertures utilisées simultanément.

Ainsi, un tel dispositif permet de mettre un procédé conforme à l'invention avec une cadence élevée.

Par ailleurs, le fait que le centre acoustique Ca d'une sous-ouverture 16 soit entouré par au moins trois centres acoustiques non alignés permet d'obtenir des données volumiques nécessaires pour être en mesure de calculer des paramètres viscoélastiques locaux tels que le module de cisaillement, la viscosité, le module d'Young, le coefficient de Poisson.

En considérant par exemple l'élasticité, ou module d'Young noté E, ce calcul peut s'effectuer à l'aide des opérations indiquées dans la demande de brevet FR 2869521 précédemment citée. De fait, l'élasticité E d'un tissu peut être calculée à partir de l'équation suivante :

$$E = 3\rho V_s^2$$

Où p est la densité du milieu et $V_s$ représente la vitesse de propagation de l'onde de cisaillement.

Dans l'hypothèse où le milieu est isotrope et linéaire, la vitesse $V_s$ de cisaillement vérifie

$$V_s = \sqrt{\frac{\partial^2 u/\partial t^2}{\Delta u}}$$

Où u est le déplacement, la déformation ou la vitesse de déformation mesurée suivant une direction donnée et $\Delta u$ est le Laplacien de u.

L'obtention des paramètres de déformations selon des axes 20 (figure 2) situés au centre et aux sommets d'un plan hexagonal 22 permet de calculer de manière exacte le Laplacien du déplacement u tandis que, contrairement aux techniques élastographiques citées précédemment, aucune hypothèse simplificatrice quant à l'expression du Laplacien n'est nécessaire, cette expression étant prise dans son intégralité.

A titre d'exemple, la discrétisation du Laplacien de u en un point i, centre de l'hexagone, peut s'écrire en fonction des valeurs de u aux sommets j de hexagone :

$$(\Delta u)^i = \frac{1}{\sqrt{3}a^2} \sum_{j=1}^{6} \frac{2}{\sqrt{3}} (u^j - u^i) + \frac{1}{b^2} (u^z + u^{-z} - 2u^i)$$

Où a et b représentent les dimensions latérales et $u^z$ et $u^{-z}$ sont les valeurs de u en élévation par rapport l'hexagone 22 considéré.

La vitesse de cisaillement peut être obtenue à partir de l'équation suivante :

$$V_s = \sqrt{\frac{\dfrac{u^{i,t+} + u^{i,t-} - 2u^{i,t}}{T^2}}{\left[\dfrac{1}{\sqrt{3}a^2} \sum_{j=1}^{6} \dfrac{2}{\sqrt{3}} (u^{j,t} - u^{i,t}) + \dfrac{1}{b^2} (u^{z+,t} + u^{z-,t} - 2u^{i,t})\right]}}, \forall t \in \left[t_{min}, t_{max}\right]$$

Où $t_{min}$ et $t_{max}$ délimitent la période de mesure.

Il apparaît ainsi que la résolution minimale d'un dispositif dépend de la distance entre les centres acoustiques des sous-ouvertures, la résolution étant d'autant plus élevée que cette distance est faible.

Typiquement, cette distance est inférieure à 3 mm, plus généralement comprise entre 1 et 3 mm, avec un dispositif de transducteur proposé dans cette demande ce qui fournit une résolution satisfaisante de 1 mm.

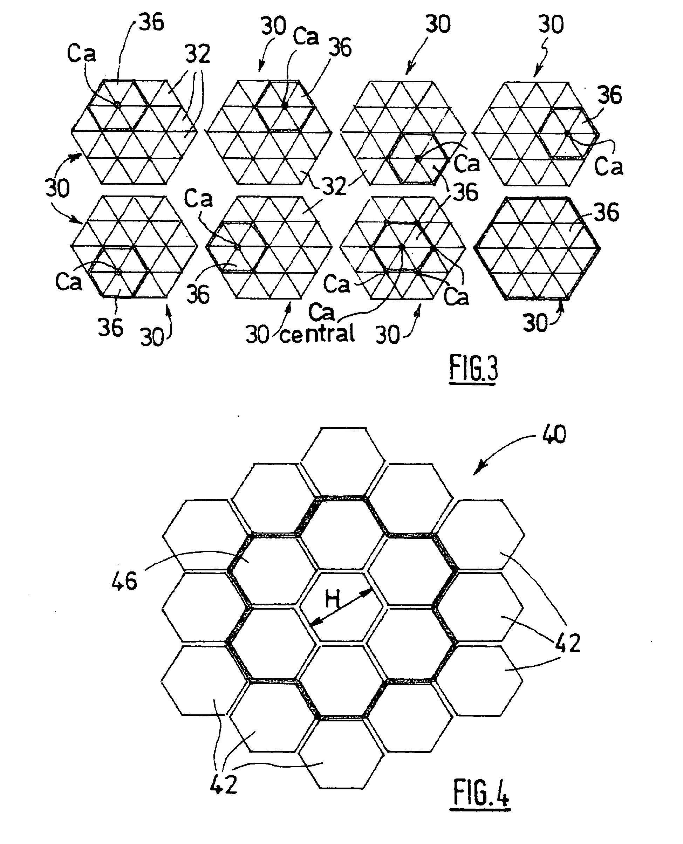

Un exemple de réalisation de l'invention est détaillée ci-dessous à l'aide de la figure 3 qui représente différentes sous-ouvertures 36, et leur centre Ca associé, formées par des éléments 32 d'un transducteur 30, ces différentes sous-ouvertures 36 étant illustrées sur différents schémas du même transducteur 30 pour des raisons de clarté.

On observe ainsi que, lors de l'acquisition des signaux ultrasonores, les centres acoustiques Ca de ces différentes sous-ouvertures 36 peut former une grille présentant un maillage 22 triangulaire, par exemple équilatéral.

Un tel maillage triangulaire présente l'intérêt de limiter la distance entre centres acoustiques Ca à la distance du côté du triangle formé par chaque élément 32. Dans cette réalisation, une telle distance est de 3 mm avec des éléments fonctionnant à une fréquence centrale de 3 MHz.

Dans ce cas, il apparaît que les sous-ouvertures 36 ont une forme hexagonale dont les côtés ont une longueur de 3 mm.

Ce transducteur 30 permet une mesure des PV à des profondeurs comprises entre 10 et 90 mm, cette profondeur étant mesurée à partir de la surface du transducteur.

Lorsqu'une sous-ouverture 36 est entièrement entourée ou délimitée par une pluralité d'autres sous-ouvertures 36, utilisées simultanément, on accroît la quantité de données relatives au volume considéré de telle sorte que la précision du calcul des PV est améliorée.

Dans cet exemple, un centre acoustique $Ca_{central}$ central peut être entouré par six centres acoustiques Ca équidistants.

L'équidistance des centres acoustiques Ca simplifie le calcul des PV en introduisant une symétrie dans la discrétisation de l'équation de propagation des ondes élastiques.

Le transducteur 30 illustré comprend 24 éléments triangulaires équilatéraux 32 et sept sous-ouvertures 36 constituées de six éléments 32 tandis qu'une sous-ouverture 36 comprend l'ensemble des éléments 32.

Une sonde équipée d'un tel transducteur ultrasonore permet d'effectuer des mesures des PV avec une profondeur qui dépend de la fréquence centrale dudit transducteur.

La mise en oeuvre d'un procédé conforme à l'invention, tel que précédemment décrit, peut s'effectuer à l'aide d'un dispositif de mesure des PV de tissus biologiques muni d'un transducteur ultrasonore comprenant des éléments dont les centres sont situés à une distance réduite, par exemple comprise entre 0,1 et 5 mm et préférentiellement entre 2 et 5 mm.

De plus, deux sous-ouvertures utilisées simultanément peuvent comprendre au moins un élément commun de façon à accroître la cadence d'acquisition des données et la cohérence temporelle des données obtenues tout en réduisant la distance entre les centres acoustiques des sous-ouvertures présentant l'élément commun.

Pour permettre une analyse volumétrique, le centre acoustique Ca d'au moins une sous-ouverture doit être préférentiellement entouré par au moins trois centres acoustiques, non alignés entre eux, correspondant à des sous-ouvertures utilisées simultanément.

Dans un second exemple, tel que décrit à la figure 4, le transducteur 40 comprend au moins 19 éléments hexagonaux 42. Dans ce cas, on peut utiliser des sous-ouvertures constituées de sept éléments telles que la sous-ouverture 46 représentée.

Dans cet exemple, les éléments hexagonaux 42 ont une hauteur H de deux millimètres et sont utilisés avec une fréquence centrale de 3,5 MHz. Dans ce cas, la résolution obtenue est de l'ordre du millimètre, cette résolution étant définie comme la dimension du plus petit volume tissulaire mesuré

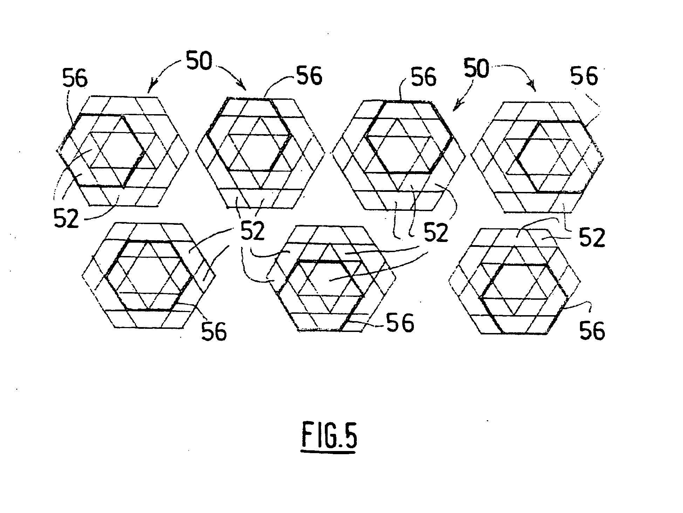

Dans un troisième exemple (figure 5) les éléments 52 ont des formes variables qui permettent néanmoins de former des sous-ouvertures 56 d'identique géométrie, à savoir hexagonale.

De fait, la présente invention est susceptible de nombreuses variantes. Elle peut être implémentée avec des éléments de différentes formes telles que : polygone (par exemple hexagonal, carré ou losange, triangle) ou circulaire, ou des combinaisons d'éléments de différentes formes.

Par ailleurs, un dispositif conforme à l'invention peut être couplé ou intégré à un système de plus grande dimension.

Par exemple un système comprenant un transducteur effectuant un traitement par hyperthermie à l'aide d'ultrasons.

Selon un autre exemple, un système comprenant des moyens pour entraîner les tissus en mouvement tels qu'un transducteur ultrasonore mettant en oeuvre la pression de radiation, terme dérivant de l'anglais « remote palpation » ou « acoustic radiation force ».

Dans un dernier exemple, un système comprenant des moyens pour entraîner les tissus en mouvement mettant en oeuvre un vibreur électromécanique.

Indépendamment de la nature des moyens mis en oeuvre pour entraîner les tissus en mouvements, une synchronisation est effectuée entre ces moyens et l'acquisition des données ultrasonores, cette acquisition pouvant comprendre le stockage de données numériques obtenues à partir des signaux électriques issus du transducteur et/ou le traitement des dites données.

Il convient de noter que les transducteurs précédemment mentionnés sont des transducteurs ultrasonores, c'est-à-dire effectuant la conversion d'une énergie électrique, respectivement ultrasonore, en une énergie ultrasonore, respectivement électrique.

Finalement, l'invention est susceptible d'être mise en oeuvre selon différentes variantes:

Dans une première variante, on répète le motif formé par les éléments d'un transducteur conforme à l'invention, par exemple tel que décrit aux figures 3, 4, 5 ou 6. Une telle répétition peut s'effectuer dans une ou plusieurs directions distinctes.

Dans une autre variante, on complète un premier motif conforme à l'invention par des éléments formant, par exemple, un second motif.

**Revendications**

1. Procède de mesure de propriétés viscoélastiques de tissus biologiques mettant en ouvre un transducteur ultrasonore (10, 30, 40, 50) muni d'éléments (12, 32, 42, 52) transformant des ondes ultrasonores réfléchies par ces tissus biologiques en signaux électriques (14), différent éléments (12, 32, 42, 52) étant groupés pour former des sous-ouvertures (16, 36, 46, 56) telles que l'acquisition des signaux (14) électriques issus des éléments (12, 32, 42, 52) d'une même sous-ouverture (16, 36, 46, 56) est effectuée simultanément, chacune de ces sous-ouvertures (16, 36, 46, 56) étant interceptée par un axe (15) de propagation d'ondes ultrasonores en un centre acoustique (Ca), **caractérisé en ce qu'**au moins un même élément (12, 32, 42, 52) appartient à au moins deux sous-ouvertures (16, 36, 46, 56) différentes, et **en ce qu'**un centre acoustique ($Ca_{central}$) est entouré par au moins trois autres centres acoustiques (Ca) non alignés.

2. Procédé selon la revendication 1 comprenant l'étape d'utiliser différentes sous-ouvertures (16, 36, 46, 56) simultanément.

3. Procédé selon la revendication 2 comprenant l'étape d'utiliser un même élément (12, 32, 42, 52) dans des sous-ouvertures utilisées simultanément.

4. Procédé selon l'une des revendications précédentes comprenant l'étape d'entraîner les tissus en mouvement.

5. Procédé selon l'une des revendications précédentes comprenant l'étape de former des sous-ouvertures (16) de telle sorte que les centres acoustiques (Ca) de ces sous-ouvertures (16) forment une grille présentant un maillage triangulaire, par exemple équilatéral.

6. Procédé selon l'une des revendications précédentes comprenant l'étape de former des sous-ouvertures (16) de telle sorte qu'une sous-ouverture (16) soit entièrement délimitée par la surface d'autres sous-ouvertures.

7. Procédé selon l'une des revendications précédentes comprenant l'étape de former les sous-ouvertures de telle sorte qu'un centre acoustique soit entouré par six centres acoustiques équidistants.

8. Dispositif comprenant des moyen pour mesurer des propriétés viscoélastiques de tissus biologiques mettant en ouvre un transducteur ultrasonore (10, 30, 40, 50) muni d'éléments (12, 32, 42, 52) transformant des ondes ultrasonores réfléchies par ces tissus biologiques en signaux électriques (14), différents éléments (12, 32, 42, 52) étant groupés pour former des sous-ouvertures (16, 36, 46, 56) telles que l'acquisition des signaux (14) électriques issus des éléments (12, 32, 42, 52) d'une même sous-ouverture (16, 36, 46, 56) est effectuée simultanément, chacune de ces sous-ouvertures (16, 36, 46, 56) étant interceptée par un axe (15) de propagation d'ondes ultrasonores en un centre acoustique (Ca), **caractérisé en ce qu'**il comprend des moyens pour qu'au moins un même élément (12, 32, 42, 52) appartienne à au moins deux sous-ouvertures (16, 36, 46, 56) différentes, et **en ce qu'**un centre acoustique ($Ca_{central}$) est entouré par au moins trois autres centres acoustiques (Ca) non alignés.

9. Dispositif selon la revendication 8 comprenant des moyens pour acquérir simultanément des signaux électriques reçus par une pluralité d'éléments regroupés en une sous-ouverture et des moyens pour effectuer la formation de voies correspondant à plusieurs sous-ouvertures simultanées présentant au moins un élément commun.

10. Dispositif selon l'une des revendications 8 ou 9 comprenant au moins 19 éléments hexagonaux ou au moins 24 éléments triangulaires équilatéraux.

11. Dispositif selon l'une des revendications 8 à 10 comprenant des éléments ayant la forme d'un polygone, par exemple un hexagone, un carré, un losange ou un triangle, ou d'un cercle.

12. Sonde (11) munie d'un dispositif conforme à 1'une des revendications 8 à 11.

13. Système (18) muni d'un dispositif conforme à l'une des revendications 8 à 11, ce système comprenant en outre des moyens pour effectuer un traitement par hyperthermie par ultrasons ou pour entraîner les tissus en mouvement.

**Claims**

1. A method of measuring the viscoelastic properties of biological tissues employing an ultrasonic transducer (10, 30, 40, 50) equipped with elements (12, 32, 42, 52) converting the ultrasonic waves reflected by these biological tissues into electrical signals (14), different elements (12, 32, 42, 52) being grouped to form sub-apertures (16, 36, 46, 56) such that the acquisition of electrical signals (14) from the elements (12, 32, 42, 52) of the same sub-aperture (16, 36, 46, 56) is carried out simultaneously, each of these sub-apertures (16, 36, 46, 56) being intercepted by an ultrasonic wave propagation axis (15) at an acoustic center (Ca), **characterized in that** at least one and the same element (12, 32, 42, 52) belongs to at least two different sub-apertures (16, 36, 46, 56) and **in that** an acoustic center ($Ca_{central}$) is surrounded by at least three other unaligned acoustic centers (Ca).

2. The method according to claim 1 comprising the step of using different sub-apertures (16, 36, 46, 56) simultaneously.

3. The method according to claim 2 comprising the step of using the same element (12, 32, 42, 52) in the sub-apertures used simultaneously.

4. The method according to one of the previous claims comprising the step of driving the tissues in movement.

5. The method according to one of the previous claims comprising the step of forming sub-apertures (16) in such a way that the acoustic centers (Ca) of these sub-apertures (16) form a grid presenting a triangular mesh, for example an equilateral triangular mesh.

6. The method according to one of the previous claims comprising the step of forming sub-apertures (16) in such a way that a sub-aperture (16) is entirely defined by the surface of other sub-apertures.

7. The method according to one of the previous claims comprising the step of forming sub-apertures in such a way that an acoustic center is surrounded by six equidistant acoustic centers.

8. A device comprising means for measuring the viscoelastic properties of biological tissues employing an ultrasonic transducer (10, 30, 40, 50) equipped with elements (12, 32, 42, 52) converting the ultrasonic waves reflected by these biological tissues into electrical signals (14), different elements (12, 32, 42, 52) being grouped to form sub-apertures (16, 36, 46, 56) such that the acquisition of electrical signals (14) from elements (12, 32, 42, 52) of the same sub-aperture (16, 36, 46, 56) is carried out simultaneously, each of these sub-apertures (16, 36, 46, 56) being intercepted by an ultrasonic wave propagation axis (15) at an acoustic center (Ca), **characterized in that** it comprises means so that at least one and the same element (12, 32, 42, 52) belongs to at least two different sub-apertures (16, 36, 46, 56), and **in that** an acoustic center ($Ca_{central}$) is surrounded by at least three other unaligned acoustic centers (Ca).

9. The device according to claim 8 comprising means for simultaneously acquiring electrical signals received by a plurality of elements grouped together in a sub-aperture and means for carrying out the formation of channels corresponding to several simultaneous sub-apertures presenting at least one common element.

10. The device according to one of claims 8 or 9 comprising at least 19 hexagonal elements or at least 24 equilateral triangular elements.

11. The device according to one of claims 8 to 10 comprising elements having the shape of a polygon, for example a hexagon, square, diamond or triangle, or a circle.

12. A probe (11) equipped with a device in conformance with one of claims 8 to 11.

13. A system (18) equipped with a device in conformance with one of claims 8 to 11, this system in addition comprising means for performing ultrasonic hyperthermia treatment or for driving tissues in movement.

**Patentansprüche**

1. Verfahren zum Messen von viskoelastischen Eigenschaften von biologischen Geweben, bei dem ein Ultraschallwandler (10, 30, 40, 50) eingesetzt wird, der mit Elementen (12, 32, 42, 52) versehen ist, die Ultraschallwellen, die von diesen biologischen Geweben reflektiert werden, in elektrische Signale (14) umwandeln, wobei verschiedene Elemente (12, 32, 42, 52) in Gruppen angeordnet sind, um Suböffnungen (16, 36, 46, 56) zu bilden, so dass die Erfassung der elektrischen Signale (14), die aus Elementen (12, 32, 42, 52) einer gleichen Suböffnung (16, 36, 46, 56) stammen, gleichzeitig durchgeführt wird, wobei jede dieser Suböffnungen (16, 36, 46, 56) von einer Ultraschallwellenausbreitungsachse (15) in einem akustischen Zentrum (Ca) geschnitten wird, **dadurch gekennzeichnet, dass** mindestens ein gleiches Element (12, 32, 42, 52) zu mindestens zwei verschiedenen Suböffnungen (16, 36, 46, 56) gehört und dass ein akustisches Zentrum ($Ca_{central}$) von mindestens drei anderen, nicht ausgerichteten, akustischen Zentren (Ca) umgeben ist.

2. Verfahren nach Anspruch 1, das den Schritt umfasst, verschiedene Suböffnungen (16, 36, 46, 56) gleichzeitig zu verwenden.

3. Verfahren nach Anspruch 2, das den Schritt umfasst, ein gleiches Element (12, 32, 42, 52) in gleichzeitig verwendeten Suböffnungen zu verwenden.

4. Verfahren nach einem der vorhergehenden Ansprüche, das den Schritt umfasst, eine Bewegung der Gewebe zu bewirken.

5. Verfahren nach einem der vorhergehenden Ansprüche, das den Schritt umfasst, Suböffnungen derart (16) zu bilden, dass die akustischen Zentren (Ca) dieser Suböffnungen (16) ein Gitter bilden, das eine Dreiecksvermaschung, zum Beispiel eine gleichseitige Dreiecksvermaschung, aufweist.

6. Verfahren nach einem der vorhergehenden Ansprüche, das den Schritt umfasst, Suböffnungen (16) derart zu bilden, dass eine Suböffnung (16) vollständig durch die Oberfläche anderer Suböffnungen begrenzt ist.

7. Verfahren nach einem der vorhergehenden Ansprüche, das den Schritt umfasst, die Suböffnungen derart zu bilden, dass ein akustisches Zentrum von sechs akustischen Zentren abstandsgleich umgeben.

8. Vorrichtung, die Mittel umfasst, um die viskoelastischen Eigenschaften von biologischen Geweben zu messen, bei dem ein Ultraschallwandler (10, 30, 40, 50) eingesetzt wird, der mit Elementen (12, 32, 42, 52) versehen ist, die Ultraschallwellen, die von diesen biologischen Geweben reflektiert werden, in elektrische Signale (14) umwandeln, wobei verschiedene Elemente (12, 32, 42, 52) in Gruppen angeordnet sind, um Suböffnungen (16, 36, 46, 56) zu bilden, so dass die Erfassung der elektrischen Signale (14), die aus Elementen (12, 32, 42, 52) einer gleichen Suböffnung (16, 36, 46, 56) stammen, gleichzeitig durchgeführt wird, wobei jede dieser Suböffnungen (16, 36, 46, 56) von einer Ultraschallwellenausbreitungsachse (15) in einem akustischen Zentrum (Ca) geschnitten wird, **dadurch gekennzeichnet, dass** sie Mittel umfasst, damit mindestens ein gleiches Element (12, 32, 42, 52) zu mindestens zwei verschiedenen Suböffnungen (16, 36, 46, 56) gehört und dass ein akustisches Zentrum ($Ca_{central}$) von mindestens drei anderen, nicht ausgerichteten, akustischen Zentren (Ca) umgeben ist.

9. Vorrichtung nach Anspruch 8, die Mittel umfasst, um gleichzeitig elektrische Signale zu erfassen, die von einer Vielzahl von Elementen empfangen werden, die in eine Suböffnung zusammengefasst sind und Mittel, um die Bildung von Wegen durchzuführen, die mehreren gleichzeitigen Suböffnungen entsprechen, die mindestens ein gemeinsames Element aufweisen.

10. Vorrichtung nach einem der Ansprüche 8 oder 9, die mindestens 19 sechseckige Elemente oder mindestens 24 gleichseitige dreieckige Elemente umfasst.

11. Vorrichtung nach einem der Ansprüche 8 bis 10, die Elemente umfasst, die die Form eines Polygons, zum Beispiel ein Sechseck, ein Quadrat, eine Raute oder ein Dreieck, oder eines Kreises aufweisen.

12. Sonde (11), die mit einer Vorrichtung gemäß einem der Ansprüche 8 bis 11 versehen ist.

13. System (18), das mit einer Vorrichtung gemäß einem der Ansprüche 8 bis 11 versehen ist, wobei dieses System ferner Mittel zum Durchführen einer hyperthermischen Behandlung mittels Ultraschall oder zum Bewirken einer Bewegung der Gewebe umfasst.

FIG.1

FIG.2

FIG.3

FIG.4

FIG.5

**EP 2 181 324 B1**

**RÉFÉRENCES CITÉES DANS LA DESCRIPTION**

**Documents brevets cités dans la description**

- FR 2869521